# EUROPÄISCHE PATENTSCHRIFT

(11) **EP 2 872 481 B1**
(45) Veröffentlichungstag und Bekanntmachung des Hinweises auf die Patenterteilung: **24.08.2016**
(21) Anmeldenummer: 13736539.1
(22) Anmeldetag: 09.07.2013
(51) Int. Cl.: C07C 263/10, C07C 263/20

(54) **VERFAHREN ZUR AUFARBEITUNG VON DESTILLATIONSRÜCKSTÄNDEN AUS DER ISOCYANATHERSTELLUNG**
METHOD FOR WORKING UP DISTILLATION RESIDUES FROM ISOCYANATE PRODUCTION
PROCÉDÉ POUR TRAITER DES RÉSIDUS DE DISTILLATION PROVENANT DE LA PRODUCTION D'ISOCYANATE

(30) Priorität: 11.07.2012 EP 12175958
(43) Veröffentlichungstag der Anmeldung: 20.05.2015
(73) Patentinhaber: Covestro Deutschland AG, 51373 Leverkusen (DE)
(72) Erfinder: STEFFENS, Friedhelm, 51373 Leverkusen (DE); LODDENKEMPER, Tim, 41542 Dormagen (DE); BELLINGHAUSEN, Rainer, 51519 Odenthal (DE); KEMPKES, Hartwig, 51491 Overath (DE)
(74) Vertreter: Levpat
(86) Internationale Anmeldenummer: PCT/EP2013/064444
(87) Internationale Veröffentlichungsnummer: WO 2014/009342

(56) Entgegenhaltungen:
- EP-A1- 0 017 972
- EP-A1- 0 626 368
- US-A- 4 216 063
- US-A- 4 918 220

## Beschreibung

Die vorliegende Erfindung betrifft ein Verfahren zur Aufarbeitung von Destillationsrückständen aus der Isocyanatherstellung, bei dem in Destillationsrückständen vorhandenes monomeres Isocyanat mittels eines Sprühtrocknungsverfahrens zurückgewonnen und so die Gesamtausbeute an monomerem Isocyanat signifikant erhöht wird.

Bei der großtechnischen Herstellung von Isocyanaten fallen Destillationsrückstände an, die der weiteren Aufarbeitung bedürfen. Diese Destillationsrückstände enthalten unter anderem polymere Produkte sowie noch Anteile an monomerem Isocyanat. Die vorliegende Erfindung betrifft ein Verfahren, das es ermöglicht, diesen Anteil an monomerem Isocyanat im Destillationsrückstand auf einfache Weise in hoher Ausbeute zurückzugewinnen.

Der Stand der Technik zur Behandlung der genannten Destillationsrückstände der Isocyanat-Herstellung beschreibt verschiedene Verfahren. Generelle Ziele der Rückstandsbehandlung sind die Maximierung der Isocyanat-Ausbeute, die Minimierung des Mengenanfalls an Rückstand und eine möglichst sinnvolle kostengünstige und einfache Verwertung der für den Isocyanat-Herstellprozess nicht mehr nutzbaren Rückstandsmenge.

Prinzipiell sind folgende Verfahren bekannt:
Grundsätzlich kann der Destillationsrückstand kontinuierlich oder diskontinuierlich verbrannt werden. Das Verfahren ist technisch einfach und kann zur Nutzdampferzeugung eingesetzt werden, sofern eine dafür geeignete Anlage zur thermischen Verwertung in der Nähe der Isocyanatproduktionsanlage existiert, um eine Entsorgung über eine Rohrleitungsanbindung zu gewährleisten. Der große Nachteil dieses Verfahrens ist jedoch der Ausbeuteverlust, dadurch verursacht, dass der Destillationsrückstand immer auch Anteile des Wertprodukts Isocyanat enthält, das mitverbrannt wird. Würde die Destillation des Isocyanats so betrieben, dass das Isocyanat aus dem Sumpf vollständig oder annähernd vollständig entfernt würde, verbliebe ein fester, nur sehr schwer zu verarbeitender Rückstand. Um dies zu vermeiden, werden die Destillationsbedingungen üblicherweise so gewählt, dass das Sumpfprodukt der Destillationskolonne flüssig bleibt, was jedoch nur gelingt, wenn dieses noch substanzielle Anteil des gewünschten Isocyanats enthält, die somit unweigerlich mit der Verbrennung zugeführt werden.

US 4,216,063 beschreibt einen kontinuierlichen Prozess zur zersetzungsfreien Abtrennung von Toluylendiisocyanat (TDI) von Destillationsrückständen, bei dem TDI in einem Verdampfer unter einem Vakuum von 1 mm Hg bis 50 mm Hg bei einer Temperatur von 210 °C bis 250 °C bei einer Mindestverweilzeit von bevorzugt 15 Minuten verdampft wird, wobei Rückstände kontinuierlich entfernt werden.

US 4,918,220 betrifft ein Verfahren zur Abtrennung von in den bei seiner Herstellung anfallenden Rückständen enthaltenem TDI im Hinblick auf seine Rückgewinnung. Das Verfahren ist dadurch gekennzeichnet. daß diese Rückstände mit einem inerten Gas im flüssigen oder überkritischen Zustand behandelt werden, gegebenenfalls in Gegenwart eines Coextraktionsmittels ausgewählt aus den Estern, den chlorierten oder nicht chlorierten aromatischen Kohlenwasserstoffen und den chlorierten aliphatischen Kohlenwasserstoffen. Das Verfahren eignet sich insbesondere zur Behandlung von in Form von Teeren vorliegenden Rückständen.

Zur Minimierung der Isocyanatausbeuteverluste kann der Destillationsrückstand in einen gerührten und beheizten Behälter überführt und mit hochsiedenden Kohlenwasserstoffen, vorzugsweise Bitumen, die unter den Destillationsbedingungen inert sind, vermischt werden, um möglichst vollständig das im Rückstand noch vorhandene freie Isocyanat abzudestillieren **(**EP0 548 685A2**).**

Der verbleibende, von Isocyanat befreite Rückstand kann als rieselfähiger Feststoff ausgetragen und einer Verbrennung zugeführt werden. Nachteile dieses Verfahrens sind neben dem Einsatz eines prozessfremden Stoffes (Bitumen) Ausbeuteverluste durch Polymerisation des Isocyanats, da der Prozess hohe Verweilzeiten bei hoher Temperatur beinhaltet.

Ein weiteres Verfahren zur Isocyanat-Rückstandsabtrennung ist gekennzeichnet durch den Einsatz von Knetertrocknern **(**EP0 626 368A1**).** In diesem Verfahren werden die oben beschriebenen beheizten und gerührten Behälter durch Knetertrockner ersetzt. Durch Einsatz von zum Beispiel Bitumen wird wie im oben genannten Beispiel der verbleibende Rückstand als rieselfähiger Feststoff erhalten der als Brennstoff zum Beispiel in Zementwerken eingesetzt werden kann. Vorteil dieses Verfahrens gegenüber dem oben genannten ist eine Ausbeuteerhöhung, als Nachteil können die erforderlichen höheren Investitionskosten gesehen werden, bedingt durch die aufwändigere Technik. Außerdem führt der Einsatz mechanisch bewegter Teile zwangsläufig zu höherem Wartungsaufwand.

EP0 699 659A2beschreibt ein Verfahren und eine Vorrichtung zur Abtrennung eines festen Rückstandes aus einer Lösung des Rückstandes in verdampfbaren Wertstoffen und/oder Lösungsmitteln unter Zusatz von bis zu 20 Gew.-% hochsiedenden Kohlenwasserstoffen, die unter Verdampfungsbedingungen der Wertstoffe inert sind, Erhitzen der Mischung auf Verdampfungstemperatur unter Vakuum, wobei die Wertstoffe verdampfen, abgezogen und kondensiert werden und der Rückstand als rieselfähiger Feststoff anfällt, wobei die Rückstandslösung auf ein bei Verdampfungstemperatur gehaltenes gerührtes Bett aus körnigem, festem Gut aufgebracht wird. Nachteilig hierbei ist der zusätzliche Einsatz von hoch siedenden Lösungsmitteln, die in einem weiteren Prozess aufgearbeitet werden müssen.

In der Patentliteratur sind auch Verfahren beschrieben, in denen Isocyanat-Destillationsrückstände chemisch umgesetzt werden, um industriell nutzbare Wertstoffe zu erhalten, wie zum Beispiel die Umsetzung von Rückstand aus der Herstellung von Toluylendiisocyanat mit Alkanolamin **(**US 5,902,459**)** oder mit Isocyanaten der Diphenylmethanreihe **(**DE42 11 774 A1**,** US 3,694,323**).**

Die Hydrolyse von Isocyanat-Destillationsrückständen mit Wasser zwecks Rückgewinnung des Ausgangsamin, insbesondere bei der Herstellung von Toluylendiisocyanat (nachfolgend TDI), ist ein bereits seit längerer Zeit bearbeitetes Gebiet und beispielsweise in US 3,128,310**,** US 3,331,876**,** GB 795,639**,** DE 27 03 313 A1 und EP 1 935 877 A1 beschrieben. In den zitierten Verfahren wird Isocyanat-Destillationsrückstand bei erhöhtem Druck und erhöhter Temperatur mit Wasser hydrolysiert. Dabei wird ein Teil des Rückstandes in das Ursprungsamin umgewandelt, das nach entsprechender Aufarbeitung wieder in den Phosgenierprozess zurückgeführt werden kann und damit zu einer Rückstandsminimierung führt. Unbefriedigend bei diesen Verfahren ist, dass ein Teil des Wertproduktes Isocyanat wieder zum Ausgangsstoff hydrolisiert und erneut phosgeniert werden muss. Dadurch wird das im Rückstand enthaltene Isocyanat zwar einer sinnvollen stofflichen Verwertung zugeführt., jedoch wäre es wünschenswert, das Isocyanat als solches aus dem Rückstand zurückgewinnen zu können.

EP 1 413 571 A1 und EP 1 371 633 A1 befassen sich mit der Optimierung der Aufarbeitung von TDI durch Einsatz einer Trennwandkolonne in der Destillation, was unter anderem eine Reduzierung des Gehalts an TDI im Sumpfprodukt zur Folge hat. Aber auch hier kann nicht verhindert werden, dass ein Isocyanat enthaltender Destillationsrückstand anfällt.

EP 0 017 972 A1 beschreibt ein Verfahren zur Abtrennung von TDI und/oder höhersiedenden Lösungsmitteln aus Destillationsrückständen, die bei der Herstellung von TDI durch Phosgenierung von Toluylendiamin entstehen, durch Eindampfen in einer Wirbelschicht bei Temperaturen von 140 bis 280 °C. Zu diesem Zweck wird der Destillationsrückstand aufgeheizt und bei Temperaturen von 50 bis 300 °C über eine Einbringvorrichtung, beispielsweise eine Zweistoffdüse oder mehrere Einstoffdüsen, in eine Wirbelschicht aus kleinen Vorlage-Partikeln bestimmter Korngrößen (0,5 bis 5000 µm) und einem Wirbelgas eingesprüht. Bei den Vorlage-Partikeln handelt es sich um bereits aufgearbeiteten, im Wesentlichen Wertstoff-freien Rückstand (vgl. bspw. S. 6, Z. 8 bis 21 und S. 7, Z. 15 bis 17 und 32 bis 36). Bei diesem Verfahren werden die mittels der Einbringvorrichtung in den Wirbelbehälter eingebrachten Tropfen des Destillationsrückstandes auf die Oberfläche der Vorlage-Partikel aufgesprüht und spreiten dort, was zur Verdampfung des Wertprodukts (TDI und/oder höhersiedendes Lösungsmittel) und zum Aufbau schalenförmige Granulate aus Wertstoff-freiem Rückstand führt. Die Verdampfung des Wertprodukts findet *auf* den Vorlage-Partikeln statt. Der aufzuarbeitende Rückstand ist bereits vor dem Einbringen in den Wirbelreaktor soweit an Isocyanat abgereichert, das er zumindest teilweise als Schmelze in die Wirbelschicht eingedüst werden muss (S. 4, Z. 20 bis 23), da eine zu hohe Viskosität den Zerstäubungsprozess behindert. Nachteilig ist, dass die Korngröße der Vorlage-Partikel aufwändig eingestellt werden muss (vgl. etwa die Führung der Partikel in der Abbildung über den Auslass (5), die "Klassiereinrichtung" (18) und die Zerkleinerungsvorrichtung (8) zurück in den Wirbelreaktor).

Ein solcher Granulationsprozess weist i. d. R. keine längeren Zykluszeiten auf und muss nach bestimmten Zeitabständen zwecks Zwischenreinigung abgefahren werden. Dies ist für den vorliegenden Fall der Aufarbeitung von Isocyanat-haltigen Rückständen aufgrund der erforderlichen Inertisierung des Reaktionsraumes und den hohen Temperaturen sowie der Anfahrproblematik nachteilig. Ein stationärer Prozess bedingt, dass sich in der Wirbelschicht wieder ein Bett mit spezifischer Korngrößenverteilung einstellt. Fehlt beispielsweise Feinanteil, so besteht die Gefahr, dass der Reaktor nach Anwachsen des ursprünglichen Bettinhaltes leer gefahren wird, während bei zu hohem Feinanteil die Ausbildung eines zu großen Bettes erfolgen und der Prozess kollabieren kann infolge des dann auftretenden hohen mechanischen Abriebes. Daher werden solche Granulationsprozesse meist genau beobachtet durch regelmäßige Probenahmen und/oder visuelle Beobachtungsmöglichkeiten. Beide Maßnahmen erfordern nicht nur einen erhöhten manuellen Betreuungsaufwand, sondern sind auch schwieriger zu realisieren aufgrund der erforderlichen Prozessbedingungen, insbesondere aufgrund der bereits oben schon erwähnten Inertisierung und hohen Temperaturen.

Infolge der vorstehend genannten Nachteile des Standes der Technik bestand ein Bedarf an einem einfachen und wirtschaftlichen Verfahren zur Gewinnung von monomerem Isocyanat aus einem in der Isocyanat-Herstellung anfallendem Destillationsrückstand. Dieses sollte sich neben einer möglichst großen Ausbeute an monomerem Isocyanat insbesondere durch Betriebsstabilität bei minimiertem Betreuungsaufwand auszeichnen.

Diesem Bedarf Rechnung tragend ist ein Gegenstand der vorliegenden Erfindung ein Verfahren zur Herstellung eines Isocyanats, umfassend die folgenden Schritte:
a) Phosgenierung eines primären Amins unter Erhalt eines das korrespondierende Isocyanat umfassenden rohen Verfahrensprodukts,
b) Aufarbeitung des unter a) erhaltenen rohen Verfahrensprodukts, wobei die Aufarbeitung mindestens einen Destillationsschritt umfasst, bei dem monomeres Isocyanat als Destillat und ein Destillationsrückstand (100) umfassend, neben nicht verdampfbaren Anteilen wie bspw. polymeren Isocyanat-Spezies, monomeres Isocyanat anfallen,
c) Aufarbeitung des unter b) erhaltenen Destillationsrückstands (100), wobei der Destillationsrückstand (100) und ein Trägergas (110) in einen Reaktor (1) eingesprüht werden, wobei der Destillationsrückstand (100) und das Trägergas (110) vertikal nach unten strömen, sodass das monomere Isocyanat teilweise bis vollständig verdampft und so ein getrockneter, von monomerem Isocyanat weitgehend bis vollständig befreiter Rückstand (120) und ein monomeres Isocyanat umfassender Strom (130) erhalten werden.

Es handelt sich bei dem erfindungsgemäßen Verfahren um ein sog. *Sprühtrocknungsverfahren.* Im Gegensatz zu *Granulationstrocknungsverfahren* (EP 0 017 972 A1) wird hier der in Schritt b) erhaltene Destillationsrückstand (100) bei Eintritt in den Reaktor (1) nicht in eine Wirbelschicht eingesprüht. Sofern im erfindungsgemäßen Verfahren überhaupt eine Wirbelschicht zum Einsatz kommt, wird diese erst nach einer signifikanten Vortrocknung des Rückstands und Entnahme des monomeres Isocyanat umfassenden Stroms (130) durchlaufen, was die weiter unten im Detail geschilderten Vorteile hat.

Nachfolgend wird die Erfindung im Detail erläutert. Dabei sind verschiedene Ausführungsformen, sofern sich aus dem Kontext nicht eindeutig das Gegenteil ergibt, beliebig miteinander kombinierbar.

Geeignete *primäre Amine* für die Durchführung von **Schritt a)** des erfindungsgemäßen Verfahrens sind insbesondere die Isomeren des Toluylendiamins (nachfolgend TDA), die Isomeren des Naphthyldiamins (nachfolgend NDA), 1,6-Hexamethylendiamin (nachfolgend HDA), die Isomeren des Isophorondiamins (nachfolgend IDPA) und die Isomeren des Diaminodicyclohexylmethans (nachfolgend H12-MDA). Besonders bevorzugt ist TDA, wobei die genaue jeweils vorliegende Isomerenzusammensetzung für das erfindungsgemäße Verfahren nicht von Belang ist. Üblicherweise umfasst TDA, welches bevorzugt eingesetzt wird, 78 Massen-% bis 82 Massen-% 2,4-TDA und 18 Massen-% bis 22 Massen-% 2,6-TDA, bezogen auf die Gesamtmasse der 2,4- und 2,6-TDA-Isomeren. Bezogen auf die Gesamtmasse des TDA machen dabei die 2,4- und 2,6-TDA-Isomeren bevorzugt in Summe von 95,0 Massen-% bis 100 Massen-%, besonders bevorzugt von 98,0 Massen-% bis 100 Massen-%, aus. Die Phosgenierung eines solchen primären Amins zum korrespondierenden Isocyanat ist grundsätzlich bekannt und kann nach einem der im Stand der Technik bekannten Verfahren durchgeführt werden. Als Beispiele seien die in den folgenden Literaturstellen beschriebenen Verfahren genannt: Ullmann's Encyclopedia of Industrial Chemistry, 5th ed. Vol. A 19, S. 390 ff., VCH Verlagsgesellschaft mbH, Weinheim, 1991, G. Oertel (Ed.) Polyurethane Handbook, 2nd Edition, Hanser Verlag, München, 1993, S. 60 ff, G. Wegener et. A1. Applied Catalysis A: General 221 (2001), S. 303 bis 335, Elsevier Science B.V., EP 1 369 412 A1, EP 1 754 698 B1 und EP 0 289 840 B1.

Bevorzugt findet die Umsetzung von primärem Amin und Phosgen in Schritt a) wie folgt statt:
Phosgen wird in einem stöchiometrischen Überschuss, bezogen auf das primäre Amin, eingesetzt. Die Phosgenierung kann in der Flüssig- und in der Gasphase durchgeführt werden. Auch eine Verfahrensweise zwischen diesen beiden Extremen ("Aerosolphosgenierung") ist denkbar. In allen Verfahrensvarianten wird ein Lösungsmittel eingesetzt, entweder um die Ausgangsstoffe darin zu lösen (Flüssigphasenphosgenierung) oder, in verdampften Zustand, als inerter Zusatzstoff bzw. als Reaktionsabbruchmedium in der sog. "Quenche" (Gasphasenphosgenierung). Bevorzugte Lösungsmittel sind chlorierte aromatische Kohlenwasserstoffe, wie beispielsweise Chlorbenzol, o-Dichlorbenzol, p-Dichlorbenzol, Trichlorbenzole, die entsprechenden Chlortoluole oder Chlorxylole, Chlorethylbenzol, Monochlordiphenyl, α- bzw. β-Naphthylchlorid, Benzoesäureethylester, Phthalsäuredialkylester, Diethylisophthalat, Toluol und Xylole. Weitere Beispiele für geeignete Lösemittel sind aus dem Stand der Technik bekannt. Wie außerdem aus dem Stand der Technik, z. B. WO-A-96/16028, bekannt, kann als Lösemittel für Phosgen ebenso das gebildete Isocyanat selbst fungieren. Besonders bevorzugte Lösungsmittel sind Chlorbenzol und Dichlorbenol, außerordentlich besonders bevorzugt ist o-Dichlorbenzol.

Beispiele für Flüssigphasenphosgenierungen sind in DE 37 44 001 C1, EP 0 314 985 A1, EP 1369 412 A1 und der dort zitierten Literatur beschrieben.

Beispiele für Gasphasenphosgenierungen sind in EP 0 570 799 A1, EP 1 555 258 A1, EP 1 526 129 A1 und DE 101 61 384 A1, sowie insbesondere für aliphatische Isocyanate in EP 0 289 840 B1 und EP 1 754 698 B 1 beschrieben. Vorteile dieses Verfahrens gegenüber der ansonsten üblichen Flüssigphasenphosgenierung liegen in der Energieeinsparung, bedingt durch die Minimierung eines aufwändigen Lösemittel- und Phosgenkreislaufs.

Das primäre Amin kann mit Phosgen in einer einstufigen oder zweistufigen oder ggf. mehrstufigen Reaktion umgesetzt werden. Dabei ist eine kontinuierliche wie auch diskontinuierliche Betriebsweise möglich.

Wird eine einstufige Phosgenierung in der Gasphase gewählt, so erfolgt die Umsetzung oberhalb der Siedetemperatur des primären Amins bevorzugt innerhalb einer mittleren Kontaktzeit von 0,05 bis 5 Sekunden und bei Temperaturen von 200 °C bis 600 °C (siehe DE 101 61 384 A1).

Bei der Phosgenierung in der Flüssigphase werden bevorzugt Temperaturen von 20 °C bis 240 °C und absolute Drücke von 1 bar bis 50 bar eingesetzt (siehe US-A-3,544,611).

In Schritt a) wird unabhängig von der genauen Verfahrensweise ein das korrespondierende Isocyanat umfassendes rohes Verfahrensprodukt erhalten. Das Isocyanat-Produkt fällt dabei üblicherweise als Gemisch aus monomeren und polymeren Spezies an. Unter *monomerem Isocyanat* wird im Rahmen dieser Erfindung das einfachste Isocyanat einer homologen Reihe verstanden. Im Fall von TDA als Ausgangsamin entspricht dieses dem Toluylendiisocyanat (nachfolgend TDI), im Fall von NDA dem Naphthyldiisocyanat (nachfolgend NDI), im Fall von HDA dem 1,6-Hexamethylendiisocyanat (nachfolgend HDI), im Fall von IDPA dem Isophorondiisocyanat (nachfolgend IDPI) und im Fall von H12-MDA dem Diisocyanatodicyclohexylmethan (nachfolgend H12-MDI). Ist das Ausgangsamin ein Gemisch aus verschiedenen Isomeren, so entspricht die Isomerenverteilung des entsprechenden Isocyanats im Wesentlichen der des Ausgangsamins.

Daneben entstehen bei der Phosgenierung eines primären Amins üblicherweise noch polymere Isocyanat-Spezies, deren Struktur nicht immer exakt bekannt ist. Üblicherweise handelt es sich um höhermolekulare Spezies, die sich formal von Polymerisationsprodukten des eingesetzten Amins durch Ersatz der Amin- durch Isocyanat-Gruppen ableiten lassen. Es ist z. B. bekannt, dass TDI bei höheren Temperaturen zur Dimerisierung (oder Polymerisierung) unter CO₂-Abspaltung neigt.

Solche und ähnliche Prozesse geben Anlass zur Bildung polymerer Isocyanat-Spezies. Diese polymeren Isocyanat-Spezies können sich zum Teil auch noch in Schritt b) bilden.

Nach der Phosgenierung in Schritt a) erfolgt in Schritt b) die Aufarbeitung dieses rohen Verfahrensproduktes. Diese geschieht bevorzugt dadurch, dass zunächst das rohe Verfahrensprodukt aus Schritt a) in einen flüssigen und einen gasförmigen Produktstrom in einer dem Fachmann bekannten Weise aufgetrennt wird. Der flüssige Produktstrom, das rohe Isocyanat, enthält im Wesentlichen Isocyanat als Gemisch aus monomeren und polymeren Spezies, das Lösungsmittel sowie einem geringen Anteil an nicht umgesetztem Phosgen. Der gasförmige Produktstrom besteht im Wesentlichen aus Chlorwasserstoffgas, stöchiometrisch überschüssigem Phosgen, weiteren Gasen, wie zum Beispiel Stickstoff und Kohlenmonoxid, und geringfügigen Mengen an Lösungsmittel. Dieser gasförmige Produktstrom wird einer weiteren Aufarbeitung zugeführt, wo in der Regel Lösungsmittel, überschüssiges Phosgen und entstandenes Chlorwasserstoffgas abgetrennt werden. Lösungsmittel und überschüssiges Phosgen werden aus wirtschaftlichen Gründen der Reaktion wieder zugeführt. Der Chlorwasserstoff kann verschiedenen Einsatzmöglichkeiten zugeführt werden, wie zum Beispiel einer Oxychlorierung von Ethylen zu Ethylendichlorid oder einem Recyclingverfahren, welches Chlor in den Isocyanatprozess wieder zurückführt. Zu diesen Recyclingverfahren zählen die katalytische Oxidation von Chlorwasserstoff, z. B. nach dem Deacon-Verfahren, die Elektrolyse von gasförmigem Chlorwasserstoff sowie die Elektrolyse einer wässrigen Lösung von Chlorwasserstoff (Salzsäure).

Der flüssige Produktstrom, das rohe Isocyanat, wird anschließend einer destillativen, im Allgemeinen mehrstufigen Aufarbeitung zugeführt, wobei gelöstes Phosgen sowie das Lösungsmittel abgetrennt werden. Diese destillative Aufarbeitung des rohen Isocyanats kann nach allgemein bekannten Methoden erfolgen. Beispiele sind in EP-A-1 413 571, US 2003/0230476 A1 (TDI), und EP 0289 840 B1 (HDI, IDPI und H12-MDI) beschrieben.

Die destillative Aufarbeitung des besonders bevorzugt nach dem erfindungsgemäßen Verfahren hergestellten TDI erfolgt bevorzugt nach einer der drei im Folgenden dargestellten Varianten:

### Variante 1

Die Variante 1 ist grundsätzlich beschrieben in Chem System's PERP Report for TDI/MDI (Chem Systems, Process Evaluation Research Planning TDI/MDI 98/99 S8, Tarrytown, N.Y., USA: Chem Systems 1999, S. 27 bis 32). Darin enthält das flüssige Reaktionsgemisch nach erfolgter destillativer Phosgenabtrennung noch einen Lösungsmittelanteil von > 50 Gew.-%, vorzugsweise 55 Gew.-% bis 65 Gew.-%. Dieses Gemisch wird der Lösungsmittelabtrennung zugeführt., wobei in einem Vorverdampfers ein Lösungsmittel-TDI-Gemisch in einer Lösungsmitteldestillationskolonne abdestilliert wird und ein flüssiger Sumpfaustrag des Vorverdampfers einer weiteren Verarbeitung, der sogenannten Rückstandsaufarbeitung zugeführt wird. Dieser Flüssigkeitsstrom enthält neben 2 Gew.-% bis 10 Gew.-% Lösungsmittel ca. 5 Gew.-% bis 20 Gew.-% Destillationssrückstand. In der Lösungsmitteldestillationskolonne wird Lösungsmittel abdestilliert und dem Prozess wieder zugeführt. Diese Destillation kann ein- oder zweistufig durchgeführt werden (US 6,803,438 B2). Das Sumpfprodukt dieser Lösungsmitteldestillation enthält neben TDI noch 15 Gew.-% bis 25 Gew.-% Lösungsmittelanteil. Dieser Strom wird in eine sogenannte Feinkolonne geleitet, in der restliches Lösungsmittel abdestilliert und das lösungsmittelfreie Sumpfprodukt einer Reinkolonne zugeführt wird, die im Unterdruck betrieben wird und das gereinigte verkaufsfähige Isocyanat TDI als Destillat liefert. Ein rückstandshaltiger Teilstrom aus dem Kolonnensumpf der Reinkolonne wird ebenfalls der Rückstandsabtrennung zugeführt. Die Aufgaben der Fein- und Reindestillationskolonnen können hier alternativ wie in US 2003/0230476 A1 beschrieben in einer Trennwandkolonne zusammengefasst werden, wobei ein Strom aus Leichtsiedern und Lösungsmittel, eine Fraktion reines TDI sowie ein TDI und höhersiedende Komponenten enthaltender Produktstrom als Sumpfprodukt erhalten wird. Der letztgenannte Produktstrom wird wiederum einer Destillationsrückstandsaufarbeitung zugeführt. Die in Chem System's PERP Report for TDI/MDI (Chem Systems, Process Evaluation Research Planning TDI/MDI 98/99 S8, Tarrytown, N.Y., USA: Chem Systems 1999, S. 27 bis 32 beschriebene Destillationsrückstandsaufarbeitung kann hervorragend durch den Schritt c) der vorliegenden Erfindung ersetzt werden.

### Variante 2

Im Gegensatz zu Variante 1 enthält in dieser Ausführungsform das flüssige Reaktionsgemisch nach erfolgter destillativer Phosgenabtrennung noch einen Lösungsmittelanteil von < 50 Gew.-%. Dieses Gemisch wird einem Vorverdampfer zugeführt, aus dem ein Lösungsmittel-Isocyanatgemisch mit einem Lösungsmittelanteil < 50 Gew.-%, in eine Destillationskolonne bevorzugt über Kopf abdestilliert wird. Diese Destillationskolonne entspricht der Feinkolonne in Variante 1. Der flüssige Sumpfaustrag des Vorverdampfers wird einer weiteren Verarbeitung, der sogenannten Rückstandsaufarbeitung zugeführt. Dieser Flüssigkeitsstrom enthält neben 2 Gew.-% bis 10 Gew.-% Lösungsmittel ca. 5 Gew.-% bis 20 Gew.-% Destillationssrückstand. Das lösungsmittelfreie Sumpfprodukt der Reinkolonne wird in die Reinkolonne geleitet, die im Unterdruck betrieben wird und das gereinigte verkaufsfähige Isocyanat TDI als Destillat liefert. Ein rückstandshaltiger Teilstrom aus dem Kolonnensumpf der Reinkolonne wird ebenfalls der Rückstandsabtrennung zugeführt. Die Aufgaben dieser Fein- und Reindestillationskolonnen können hier alternativ wie in EP 1 413 571 A1 beschrieben in einer Trennwandkolonne zusammengefasst werden, wobei ein Strom aus Leichtsiedern und Lösungsmittel, eine Fraktion reines TDI sowie ein TDI und höhersiedende Komponenten enthaltender Produktstrom als Sumpfprodukt erhalten wird. Der letztgenannte Produktstrom wird wiederum einer Destillationsrückstandsaufarbeitung zugeführt. Auch in Variante 2 kann die Destillationsrückstandsaufarbeitung hervorragend gemäß Schritt c) der vorliegenden Erfindung durchgeführt werden.

### Variante 3

Die Variante 3 umfasst die in den Varianten 2 und 1 beschriebenen Destillationssequenzen, jedoch ohne den jeweils erwähnten Vorverdampfer, der einen flüssigen Sumpfaustrag mit ca. 5 Gew.-% bis 20 Gew.-% Destillationsrückstand einer Rückstandsaufarbeitung zuführt. In diesem Fall wird der Anteil an Destillationsrückstand in den beschriebenen Destillationssequenzen über die flüssigen Mengenströme bis zur jeweiligen letzten TDI Reinigungskolonne mitgeführt. Dieses Verfahren ist prinzipiell ebenfalls bekannt (EP 1 717 223 A2). In diesem Fall erfolgt der komplette Austrag des Destillationsrückstands (Gemisch enthaltend Toluylendiisocyanat und Destillationsrückstand) zur Rückstandsaufarbeitung aus der letzten Destillationskolonne. Auch in Variante 3 kann die Destillationsrückstandsaufarbeitung hervorragend gemäß Schritt c) der vorliegenden Erfindung durchgeführt werden.

All den bekannten Verfahren zur destillativen Reinigung des rohen Isocyanats in Schritt b) ist gemeinsam, dass neben dem gewünschten gereinigten monomeren Isocyanat aus der Destillation ein Sumpfprodukt erhalten wird, das noch monomeres Isocyanat, also Wertprodukt, enthält. Dieses Sumpfprodukt kann entweder direkt (d. h. der Destillationsrückstand (100) ist identisch mit dem Sumpfprodukt), oder bevorzugt nach einer Aufkonzentrierung in einem geeigneten Vortrockungsapparat, der erfindungsgemäßen Rückstandsaufarbeitung in Schritt c) zugeführt werden. In der Aufkonzentrierung werden eine Gasphase umfassend monomeres Isocyanat und der Destillationsrückstand (100), der ebenfalls noch monomeres Isocyanat enthält, als flüssige Phase erhalten. Geeignete Vortrocknungsapparate sind Dünnschichtverdampfer, Kletterverdampfer, Fallfilmverdampfer, Langrohrverdampfer, Wendelrohrverdampfer, Zwangsumlaufentspannungsverdampfer und Schaufeltrockner oder eine Kombination dieser Apparate. Bevorzugt ist ein Schaufeltrockner, besonders bevorzugt ein Schaufeltrockner ohne Kühlzone und mit Austragsschnecke für den Destillationsrückstand (100). Die optionale Aufkonzentrierung in einem Vortrocknungsapparat wird nur soweit durchgeführt, dass der verbleibende Destillationsrückstand (100) noch fließfähig bleibt. Er enthält daher trotz dieses Aufkonzentrierungsschrittes noch substanzielle Mengen an monomerem Isocyanat.

Die Aufarbeitung dieses Destillationsrückstandes 100 umfassend, neben nicht verdampfbaren Anteilen wie bspw. polymeren Isocyanat-Spezies, monomeres Isocyanat, ist Gegenstand von **Schritt c)** des erfindungsgemäßen Verfahrens. In diesem Schritt wird der in Schritt b) erhaltene Destillationsrückstand (100) zusammen mit einem Trägergas (110) in einen Reaktor eingesprüht, wobei der Destillationsrückstand (100) und das Trägergas (110) vertikal nach unten strömen, sodass das monomere Isocyanat teilweise bis vollständig verdampft und so ein getrockneter, von monomerem Isocyanat weitgehend bis vollständig befreiter Rückstand (120) und ein monomeres Isocyanat umfassender Strom (130) erhalten werden.

Geeignete *Trägergase* sind dabei solche, die mit dem abzutrennenden monomeren Isocyanat unter den vorliegenden Bedingungen nicht reagieren, also inert sind. Bevorzugt sind Stickstoff, Edelgase (wie Ar, He u. a.) und Kohlenstoffdioxid. Stickstoff ist besonders bevorzugt, da er hinreichend inert und preisgünstig ist. Grundsätzlich möglich, wenn auch nicht bevorzugt, ist auch die Verwendung von Dämpfen inerter Lösungsmittel (bevorzugt ausgewählt aus Chlorbenzol, ortho-Dichlorbenzol, Toluol, Dichlormethan, Chloroform, Cyclohexan, Hexan, Xylol, Isooctan, Aceton, Tetrahydrofuran, Dioxan oder Gemischen der vorgenannten Lösungsmittel, wobei Chlorbenzol und ortho-Dichlorbenzol besonders bevorzugt sind) als Trägergas.

Als *Reaktoren* kommen in Schritt c) aus dem Stand der Technik grundsätzlich bekannte sog. Sprühtürme in Frage. Diese sind bspw. in Kröll: Trocknungstechnik, 2. Brand, 2.Aufl., 1978, Seite 275-313 beschrieben, sind üblicherweise zylindrisch aufgebaut und können einen Durchmesser von 2 m bis 10 m bei einer Höhe von 5 m bis 30 m aufweisen. Hiervon abweichende Maße sind jedoch auch denkbar. Bevorzugt weist der eingesetzte Reaktor am unteren Ende (wo der an monomerem Isocyanat abgereicherte Destillationsrückstand ausgeschleust wird) eine konusförmige Verjüngung auf.

Das Einsprühen des Destillationsrückstandes in den Reaktor kann mit üblichen Zerstäuberorganen erfolgen, wie bspw. Druckdüsen, Ein- oder Zweistoffdüsen oder Rotationszerstäubern. Bevorzugt erfolgt die Versprühung derart, dass Tropfengrößen in geeigneter Größenverteilung entstehen, die den Qualitätsanforderungen genügen und im Sprühturm ausreichend lange verweilen können, so dass eine vollständige Verdampfung von Lösungsmittelresten erreicht wird. Das heiße Trägergas, das die dazu erforderliche Wärme bereitstellt, wird je nach verwendetem Zerstäubungsorgan unterschiedlich zugeführt:
a) Im Falle von Düsen wird das Trägergas über Siebböden zugeführt, um eine gleichmäßige Verteilung des Gases zu erreichen. Ggf. kann die Heißgasverteilung durch einen über Leitbleche oder über einen dezentralen Einlass am Umfang des zylindrischen Reaktors erzeugten Drall weiter verbessert oder angepasst werden.
b) Bei Einsatz von Rotationszerstäubern wird das heiße Trägergas zentral über einen Ringspalt oberhalb des Zerstäubers zugeführt und die Gasverteilung wie auch die Verteilung der Tropfen im Turm durch Anpassen des Dralls an den Heißluftschaufeln eingestellt. Da die Gasgeschwindigkeiten bei homogener Verteilung des Gases über den Turmquerschnitt sehr klein sind, bevorzugt 0,1 m/s bis 1m/s, besonders bevorzugt 0,2 m/s bis 0,5 m/s, und die Tropfen aus den Zerstäuber mit deutlich höheren Geschwindigkeiten austreten (bevorzugt 20 m/s bis 200 m/s), ist die Gasverteilung und die Verteilung der Sprühtropfen wie oben beschrieben aufeinander abzustimmen.

Auf diese Weise wird der Destillationsrückstand in Form feiner Tröpfchen in den Reaktor verdüst. Der mittlere Tröpfchendurchmesser beträgt dabei bevorzugt von 1 µm bis 500 µm, besonders bevorzugt 20 µm bis 300 µm. Die Sprühtropfen werden so eingestellt, dass im Reaktor eine möglichst vollständige Verdampfung des monomeren Isocyanats erreicht wird. Laborversuche mit einem zylindrischen Reaktor (sprühturm) mit konusförmiger Verjüngung am unteren Ende haben gezeigt, dass bei Tropfen mit einem mittleren Durchmesser von 20 µm und einer Höhe des zylindrischen Teils des Reaktors von 1 m Höhe sowie 0,8 m Konuslänge und 0.8 m Durchmesser des zylindrischen Teils des Reaktors eine ausreichende Trocknung erreicht wird. Im Produktionsmaßstab mit typischerweise 10 bis 30 m langen Reaktoren können auch größere Tropfen mit einem mittleren Durchmesser von etwa 100 bis 300 µm getrocknet werden.

Die Temperatur des Destillationsrückstandes beim Einsprühen in den Reaktor muss mindestens so groß sein, dass die Viskosität des Rückstandes ausreichend klein ist, um eine Zerstäubung durchführen zu können. Dazu muss erfahrungsgemäß eine Viskosität unterhalb 1000 mPas, vorzugsweise unter 300 mPas und besonders bevorzugt unter 100 mPas vorliegen. Weitere Angaben zur Viskosität folgen unten. Ferner sollte die Temperatur des Rückstandes beim Einsprühen in den Reaktor unterhalb der Siedetemperatur des monomeren Isocyanats sein, um eine Verdampfung vor oder während der Zerstäubung zu verhindern. Flashverdampfungen nach oder während der Zerstäubung können zwar die Trocknungszeiten verkürzen, können aber auch eine Verfestigung der Tropfen vor der Tropfenausbildung bewirken und dadurch den Zerstäubungsvorgang behindern. Daher wird die Temperatur des Destillationsrückstands beim Einsprühen in den Reaktor bevorzugt auf einen Wert von 20 °C bis 300 °C, besonders bevorzugt von 50 °C bis 200 °C, außerordentlich besonders bevorzugt von 80 °C bis 180 °C, eingestellt, und die Temperatur des Trägergases beim Einsprühen in den Reaktor wird je nach der für die Temperatur des Destillationsrückstandes beim Einsprühen in den Reaktor gewählten Temperatur bevorzugt auf einen Wert von 150 °C bis 500 °C, besonders bevorzugt von 180 °C bis 350 °C, außerordentlich besonders bevorzugt von 220 °C bis 280 °C, eingestellt. Unter diesen Bedingungen lässt sich der Destillationsrückstand am vorteilhaftesten versprühen.

Zur Zerstäubung können wie oben erwähnt Düsen- oder Rotationszerstäuber eingesetzt werden. Zur Erzielung von staubarmen Rückständen werden bevorzugt Druckdüsen eingesetzt, bei denen der zu zerstäubende Destillationsrückstand (100) einen absoluten Druck von 1,0 bar bis 300 bar, bevorzugt von 3,0 bar bis 100 bar, besonders bevorzugt von 5,0 bar bis 20 bar, aufweisen sollte. Bei Zweistoffdüsen und auch bei Rotationszerstäubern muss häufig ein höherer Feinkornanteil hingenommen werden. Ferner ist auch eine Eindüsung von Trennmitteln (siehe unten) um die Sprühtropfen herum schwieriger. Aus diesen Gründen sind Druckdüsen besonders bevorzugt. Geeignete Druckdüsen sind dem Fachmann an sich bekannt und bspw. beschrieben in Kröll: Trocknungstechnik, 2. Baud, 2.Aufl., 1978, Seite 282-288*.*

Bevorzugt weist der Destillationsrückstand beim Einsprühen in den Reaktor bei der jeweiligen Temperatur eine Viskosität von 1 mPa s bis 1000 mPa s, besonders bevorzugt von 1 mPa s bis 300 mPa s auf. Maßgeblich sind dabei die mit einem Rheometer mit Doppelspaltgeometrie (DG24/27) bei 25 °C im Scherratenbereich von 1 bis 100 s⁻¹, Standard: 90 s⁻¹, erhaltenen Viskositätswerte. Nach Einfüllen der Proben in die Messgeometrie werden die Messungen nach einer Equilibrierzeit von 120 s gestartet. Im Scherratenbereich von 1 bis 100 s⁻¹ werden 22 Messpunkte in logarithmischer Verteilung mit einer konstanten Messpunktdauer von 10 s aufgenommen. Bevorzugt ist der Einsatz eines Bohlin CVO Rheometers. Diese Viskosität wird erforderlichenfalls eingestellt durch Erhöhung der Temperatur in den zuvor genannten Grenzen und/oder durch Vermischen des Destillationsrückstandes mit Lösungsmittel, bevorzugt Prozesslösungsmittel (d. h. das gleiche Lösungsmittel wie in Schritt a)), oder monomerem Isocyanat. Falls monomeres Isocyanat zum Verdünnen eingesetzt wird, ist der Rückgewinn an monomeren Isocyanat im Seitenabzug des Reaktors regelmäßig größer als der Einsatz desselben zur Verdünnung des Destillationsrückstands, sodass insgesamt die Ausbeute an monomerem Isocyanat gesteigert werden kann. Bevorzugt ist es jedoch, die Destillationsbedingungen in Schritt b) so zu wählen, dass der Destillationsrückstand die Destillationskolonne bereits mit der gewünschten Viskosität verlässt.

Durch die Verdampfung des monomeren Isocyanats werden die Tropfen des Destillationsrückstands getrocknet. Dabei werden klebrige, oligomere Bestandteile des ursprünglichen Destillationsrückstands so durchpolymerisiert, dass insgesamt ein fester, rieselfähiger Rückstand verbleibt, der am unteren Ende des Reaktors aus diesem ausgeschleust werden kann.

In einer bevorzugten Ausführungsform werden der das monomere Isocyanat umfassende Strom 130 in einem Seitenabzug des Reaktors 1 und der Rückstand 120 unterhalb dieses Seitenabzugs aus dem Reaktor 1 ausgeschleust. Hierbei ist es weiterhin bevorzugt, den ausgeschleusten Strom 130 in einem Abscheider (z. B. ein Zyklon) von Feinstaub zu befreien. Gegenüber der gemeinsamen Ausschleusung des Rückstands 120 und des monomeres Isocyanat umfassenden Stroms 130 unten aus dem Reaktor 1 mit anschließender Trennung dieser Ströme in einem Abscheider (z. B. Zyklon oder Filter), die grundsätzlich auch möglich ist, hat diese Verfahrensweise den Vorteil, dass im nachgeschalteten Abscheider ein zusätzlicher Feststoffaustrag aus i.d.R. feinerem Material gewonnen wird, der ggf. als Trennmittel in den Turm (Reaktor 1) zurückgefahren werden kann, s.u. für weitere Details.

In einer bevorzugten Ausführungsforn wird ein Trennmittel zugesetzt, um die Bildung nicht klebriger getrockneter Rückstandspartikel zu erleichtern. Solche Trennmittel können Zusatzstoffe sein, die bei der Temperatur im Reaktor nicht klebrig sind, also bei Aufeinandertreffen nicht zu größeren Agglomeraten verkleben, mit dem Isocyanat nicht reagieren und die Polymerisationsreaktion der oligomeren Bestandteile des ursprünglichen Destillationsrückstands nicht behindern. Bevorzugt ist das Trennmittel ausgewählt aus der Gruppe bestehend aus Talkum, Kreide, anorganischen Pigmenten und vollständig durchpolymerisiertem Rückstand. Unter den *anorganischen Pigmenten* sind insbesondere Titandioxid, Eisenoxide, Aluminiumoxid und andere Metalloxide bevorzugt. *Vollständig durchpolymerisierter Rückstand* meint den getrockneten Anteil des ursprünglichen Destillationsrückstandes. Dieser kann bspw. aus Staubabscheidem (Zyklone, Filter, siehe unten) oder durch Mahlen des am unteren Ende des Reaktors ausgetragenen Rückstands gewonnen werden. Das Trennmittel wird entweder mit dem flüssigen Destillationsrückstand vor dessen Einfuhr in den Reaktor vermischt oder es wird direkt am oberen Ende des Reaktors über eine Zuführleitung, die neben der Zuführleitung des Destillationsrückstandes angebracht ist, in den Reaktor eingebracht, wo es unmittelbar in den Tropfenstrom gelangt und mit diesem koalesziert oder sich auf der Oberfläche der vorgetrockneten Topfen absetzt. In beiden Fällen wird das Trennmittel gemeinsam oder in unmittelbarer Nähe mit dem Destillationsrückstand in den Reaktor eingebracht und soll eine Agglomeration der Sprühpartikel verhindern, im Gegensatz zu Granulationsverfahren, bei denen Feststoffpartikel in einer Wirbelschicht im Reaktor vorgelegt werden, bevor der Destillationsrückstand eingebracht wird, sodass sich der eingedüste Destillationsrückstand auf diesen Feststoffpartikeln niederschlägt und größere Teilchen wachsen.

In einer weiteren bevorzugten Ausführungsform wird bei der Variante mit Entnahme des Stroms 130 im Seitenabzug die Bildung nicht klebriger getrockneter Rückstandspartikel erleichtert, indem der nach unten fallende Rückstand **nach** Abzug des monomeren Isocyanats im Seitenabzug durch ein Wirbelbett geführt wird, ehe er unten aus dem Reaktor 1 ausgeschleust wird. Der Rückstand ist, wenn er auf das Wirbelbett trifft, schon weitgehend vorgetrocknet, d. h. bevorzugt 50 % bis 99 % des mit dem Destillationsrückstands 100 in den Reaktor 1 eingebrachten monomeren Isocyanats sind bereits verdampft und wurden über den Seitenabzug 130 aus dem Reaktor 1 entfernt. Die Abmessungen des Reaktors 1, die Temperatur des Trägergases 110 und die des Destillationsrückstandes 100 beim Einsprühen in den Reaktor sowie die Strömungsgeschwindigkeiten werden in dieser Ausführungsform bevorzugt so aufeinander abgestimmt, dass die genannten Werte an verdampften monomerem Isocyanat erreicht werden.

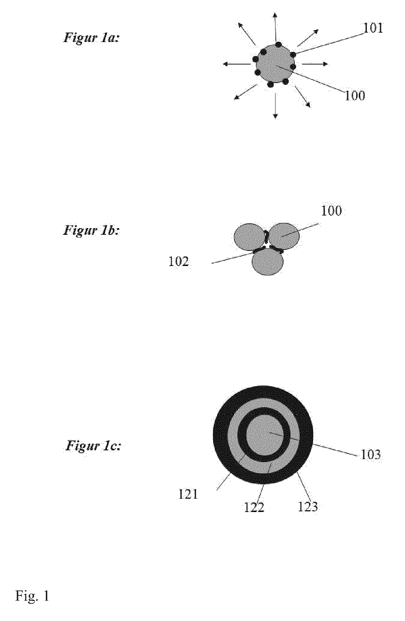

Das Wirbelbett wird bevorzugt aus den gleichen Feststoffen inkl. des zuvor als Trennmittel bezeichneten Feststoffes und dem gleichen Gas, das als Trägergas (110) eingesetzt wird, gebildet. Das Trägergas *für die Wirbelschicht,* (nachfolgend auch Fluidisiergas genannt) wird von unten, unterhalb der Wirbelschicht, in den Reaktor zugeführt und wirbelt die vorgetrockneten Partikel auf. Dadurch wird für die komplette Trocknung und Polymerisation des Destillationsrückstandes eine längere Verweilzeit erreicht. Dadurch, dass im erfindungsgemäßen Verfahren der Destillationsrückstand bei Eintritt in das Wirbelbett schon weitgehend vorgetrocknet ist und das Wirbelbett unterhalb des Seitenabzugs für das monomere Isocyanat (130) angeordnet ist, wird erreicht, dass die Rückstandspartikel nicht durch Spreiten von noch flüssigem Rückstand zwdebelförmig aufgranulieren, sondern lediglich durch Zusammenkleben einzelner Partikel aufagglomerieren und dadurch staubärmere Pulver aus durchgetrocknetem Rückstand entstehen. Diese Agglomeration ist für den Prozess nicht zwingend erforderlich, da die in die Wirbelschicht eintretenden Partikel bereits in einer Größenordnung sind, mit der eine Wirbelschicht betrieben werden kann und nicht oder nur zu einem geringen Teil mit dem Fluidisiergas ausgetragen werden. Ggf. muss die Agglomeration durch Zugabe von Trennmitteln begrenzt werden, damit nicht zu große Agglomerate entstehen, die sich auf dem Anströmboden der Wirbelschicht ablagern. Die Unterschiede zwischen den Trocknungsverfahren sind schematisch in Figur 1 dargestellt:
Figur la (erfindungsgemäß) zeigt schematisch ein trocknendes Rückstandsteilchen 100, das von Trennmittelteilchen 101 bedeckt ist, die ein Zusammenkleben verhindern oder zumindest zurückdrängen. Die Verdampfung flüchtiger Bestandteile wird durch die Pfeile symbolisiert.
Figur lb (erfindungsgemäß) zeigt schematisch ein staubarmes Agglomerat, das erhalten wird, wenn der *vorgetrocknete* Rückstand durch eine Wirbelschicht aus Feststoffteilchen 102 geführt wird.
Figur 1c (nicht erfindungsgemäß, Trocknung mittels Granulation) zeigt schematisch den zwiebeiförmigen Aufbau eines mittels Granulation getrockneten Rückstandsteilchen. Im Kern befindet sich ein Feststoffpartikel 103, der in einer Wirbelschicht, in die *der noch nicht vorgetrocknete* Rückstand eingesprüht wurde, vorgelegt wurde. Häufig bestehen diese Feststoffpartikel aus vollständig durchpolymerisiertem Rückstand. Um den Kern befinden sich zwiebelförmig Schichten 121, 122, 123, etc. aus getrocknetem Rückstand.

Im erfindungsgemäßen Verfahren wird der verfestigte, von monomerem Isocyanat weitgehend bis vollständig befreite Destillationsrückstand 120 am unteren Ende des Reaktors, das bevorzugt konusförmig ist, durch geeignete, dem Fachmann an sich bekannte, Austragsvorrichtungen ausgeschleust. Solche Austragsvorrichtungen sind bevorzugt Zellradschleusen. Im Falle der Ausführungsform mit angeschlossener Wirbelschicht am Boden des Sprühturmes erfolgt die Entnahme des Rückstandes seitlich über ein Wehr.

Das abgetrennte monomere Isocyanat verlässt den Reaktor bevorzugt über einen Seitenabzug zusammen mit dem Großteil des Trägergasstroms. In der weniger bevorzugten Ausführungsform des gemeinsamen Austrags von Rückstand und monomeren Isocyanats aus dem Reaktor 1 werden alle Gasströme (Trägergas und verdampftes monomeres Isocyanat) und die Rükstandpartikel gemeinsam unten aus dem Reaktor über einen Konus ausgetragen und anschließend in einem Abscheider (Zyklon oder Filter) voneinander in die Ströme 120 und 130 getrennt. Die weitere Aufarbeitung dieser Ströme erfolgt dann ebenso wie in der bevorzugten Ausführungsform der Abnahme von Strom 130 in einem Seitenabzug.

Zur Abscheidung von evtl. mitgerissenen Feststoffpartikeln wird der Strom aus monomerem Isocyanat und Trägergas (130) bevorzugt durch ein Zyklon oder Filter geleitet. Die dort abgetrennten Feststoffanteile werden entweder mit dem Rückstand 120 vermischt und gemeinsam weiter verarbeitet, oder sie werden, wie oben geschildert, als Trennmittel eingesetzt. Der Strom aus monomerem Isocyanat und Trägergas wird nun möglichst rasch abgekühlt, um eine Polymerisation des monomeren Isocyanats zu verhindern. Dies geschieht bevorzugt durch einen oder mehrere Wärmeaustauscher, in denen die Temperatur des Gasstroms von einer Temperatur von 150 °C bis 250 °C (Austrittstemperatur bei Verlassen des Reaktors) auf einen Wert von 50 °C bis 100 °C abgekühlt wird. Neben der Abkühlung in einem Wärneaustauscher ist es auch möglich, den Strom aus Trägergas und monomerem Isocyanat durch Eindüsen eines inerten Lösungsmittels oder durch Einleiten in ein inertes Lösungsmittel abzukühlen. Dabei wird bevorzugt das gleiche Lösungsmittel eingesetzt wie in Schritt a).

Das auf diese Weise auskondensierte monomere Isocyanat wird bevorzugt teilweise bis vollständig, bevorzugt vollständig, mit dem in Schritt b) als Destillat erhaltenem monomeren Isocyanat vereinigt und der weiteren Anwendung zugeführt. Sofern das in Schritt c) erhaltene monomere Isocyanat noch Lösungsmittel umfasst (ggf. zur Einstellung einer geeigneten Viskosität oder zur Abkühlung zugegeben), kann dieses natürlich zuvor abgetrennt werden.

Die Verweilzeit von monomerem Isocyanat von Eintritt in den Reaktor von Schritt c) bis zum Abkühlen nach Verlassen des Reaktors beträgt bevorzugt von 1 s bis 100 s, besonders bevorzugt von 3 s bis 60 s und außerordentlich besonders bevorzugt von 10 s bis 30 s. Die gewünschte Verweilzeit wird dabei auf die zur Verdampfung des monomeren Isocyanats erforderliche Verweilzeit abgestimmt, insbesondere auf die maximale Verweilzeit, die für die Tropfen mit den größeren Durchmessern erforderlich ist. Diese Verweilzeit wird durch Wahl/Auslegung der Reaktorlänge realisiert, abgestimmt auf die Gas- und Sinkgeschwindigkeiten der Tropfen. Je geringer die Temperatur und je kürzer die Verweilzeit, desto geringer ist die Gefahr einer Polymerisation des monomeren Isocyanats. Andererseits sind für eine möglichst vollständige Verdampfung des monomeren Isocyanats aus dem Destillationsrückstand natürlich eine bestimme Mindesttemperatur und Mindestverweilzeit erforderlich, sodass die tatsächlich eingestellten Parameter einen Kompromiss zwischen diesen beiden Anforderungen widerspiegeln. Ein Vorteil des vorliegenden Verfahrens ist darin zu sehen, dass infolge der Verdampfungskühlung die Sprühtropfen eine deutlich niedrigere Temperatur haben als das sie umgebende Trägergas, wodurch die Polymerisationsneigung weiter zurückgedrängt wird.

Die bei der Kondensation des monomeren Isocyanats anfallende Gasphase, die im Wesentlichen das Trägergas umfasst, wird bevorzugt, über ein Kreisgasgebläse und nach Aufheizung auf die gewünschte Reaktoreintrittstemperatur in den Trocknungsprozess zurückgeführt. Ferner wird vor der Aufheizung und Rückführung ein Teil des wiedergewonnenen Trägergases ausgeschleust und ggf. durch frisches ersetzt, um nicht kondensierbare Gase, die bei der Verdampfung und Teilpolymerisation entstehen können, aus dem Prozess auszuschleusen und um ggf. eine Anreicherung von in größerer Menge störenden Komponenten (z. B. Gasanteile im Rückstand oder aus Zersetzungs-/Polymerisationsreaktionen, Abreinigungsgas aus der Filterabreinigung, Falschlufteinträge, z. B. aus Zellenradschluesen.) zu vermeiden. Die Abführung dieses kleinen Abgasstromes aus dem Prozess erfolgt bevorzugt nach dem Kreisgasgebläse, um den Druckaufbau bei der Ventilation zu nutzen und den kleinen Abgasstrom ohne weitere Transport-/Druckaufbauaggregate (Ventilator, Injektor o. ä.) vornehmen zu können.

Zur Steigerung der Verfügbarkeit des Verfahrens werden in einer bevorzugten Ausführungsform in allen Aggregaten sog. CIP- (CleanInPlace) Reinigungssysteme empfohlen, die durch gezieltes lokales Eindüsen von Reinigungsflüssigkeit und Auffangen der Reinigungsflüssigkeit über verschließbare Abläufe und einen Auffangbehälter, aus dem die Pumpe zur Verdüsung gespeist wird, eine Vorort-Reinigung der Aggregate ohne Aufhebung der Inertisierung und manuelles Öffnen der Apparate erlaubt. Von diesem Prinzip kann im Kondensator bereits Gebrauch gemacht werden, indem er als Wäscher ausgeführt und Lösungsmittel verdüst wird.

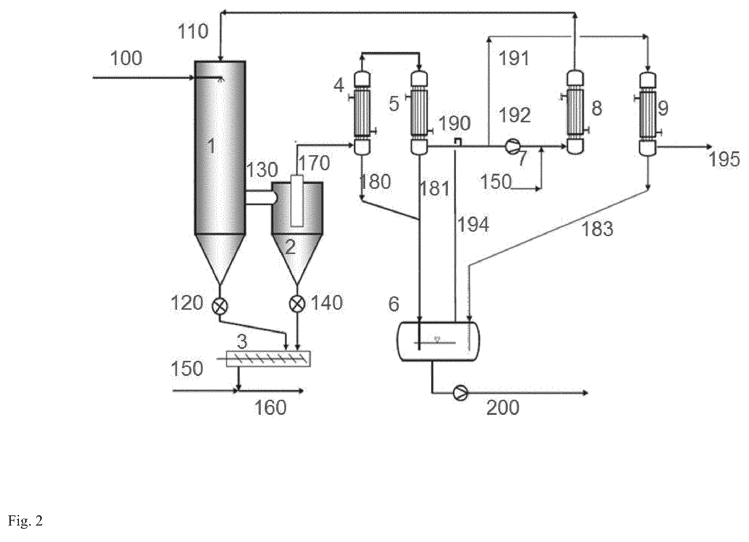

Figur 2 veranschaulicht eine mögliche Ausgestaltung des erfindungsgemäßen Verfahrens am Beispiel eines aufzuarbeitenden Destillationsrückstandes 100, der aus einer TDI-Produktionsanlage stammt und 50 Massen-%, bezogen auf die Gesamtmasse von 100, monomeres TDI enthält:
Der aufzuarbeitende Destillationsrückstand 100 mit einer Temperatur von 150 °C wird mit 2000 kg/h in den Sprühturm 1 am oberen Ende desselben eingebracht, ebenso wie das Trägergas 110, welches eine Temperatur von 300 °C hat und unter einem absoluten Druck von 1200 mbar steht. Der durchpolymerisierte Rückstand verlässt den Sprühturm 1 am unteren Ende des Konus als Strom 120 mit einer Temperatur von 250 °C. Das monomere Isocyanat (TDI) ist im Seitenabzugsstrom 130 enthalten. Dieser wird in einem Zyklon 2 von mitgerissenen Staubanteilen befreit. Letztere werden als Strom 140 ausgetragen und zusammen mit 120 in einer Kühlschnecke 3 abgekühlt. Der Austrag aus der Kühlschnecke wird mit Stickstoff (150) beaufschlagt und als Strom 160, der eine Temperatur von 60 °C hat, mit 1050 kg/h der weiteren Verwendung (z. B. als Brennstoff) zugeführt. Die im Zyklon 2 erhaltene Gasphase 170 mit einer Temperatur von 250 °C wird in zwei Kondensatoren 4 und 5 sukzessive auf zunächst 150 °C (4) und schließlich 50 °C (5) abgekühlt. Die jeweils erhaltenen Flüssigphasen 180 und 181, welche das monomere Isocyanat (TDI) und ggf. Lösungsmittel enthalten, werden zusammengeführt und in den Kondensatsammelbehälter 6 geleitet. Der in 4 und 5 nicht kondensierte Strom 190 wird aufgeteilt: Ein Teilstrom 191 wird durch einen weiteren Kühler 9 geleitet und dort auf 30 °C abgekühlt, die so erhaltene Flüssigphase 183 wird ebenfalls dem Kondensatsammelbehälter 6 zugeführt. Die Gasphase des Koudensatsammelbehälters steht über Leitung 194 in Kontakt mit 190. Nicht kondensierbare Anteile 195 werden als Purge-Strom aus dem Verfahren ausgetragen. Der Teilstrom 192 wird, nach Durchlaufen des Kreisgasgebläses 7 und Anreicherung mit Stickstoff 150, im Kreisgasheizer 8 auf die angestrebte Reaktoreintrittstemperatur aufgeheizt, um so den Trägergasstrom 110 bereitzustellen. Das Wertprodukt, Strom 200 (950 kg/h), setzt sich aus den Teilströmen 181 bis 183 zusammen. Je nach der genauen Ausgestaltung des Verfahrens enthält Strom 200 neben dem monomeren Isocyanat (TDI) noch Anteile an Lösungsmittel. Je nach der geplanten Verwendung wird dieses nach bekannten Verfahren abgetrennt. Bevorzugt wird Strom 200 jedoch direkt dem in Schritt b) erhaltenem Destillat an monomerem Isocyanat zugeschlagen und mit diesem gemeinsam weiter aufgearbeitet.

## Patentansprüche

1. Verfahren zur Herstellung eines Isocyanats, umfassend die folgenden Schritte:
a) Phosgenierung eines primären Amins unter Erhalt eines das korrespondierende Isocyanat umfassenden rohen Verfalwensprodukts,
b) Aufarbeitung des unter a) erhaltenen rohen Verfahrensprodukts, wobei die Aufarbeitung mindestens einen Destillationsschritt umfasst, bei dem monomeres Isocyanat als Destillat und ein Destillationsrückstand (100) umfassend monomeres Isocyanat anfallen,
c) Aufarbeitung des unter b) erhaltenen Destillationsrückstands (100), wobei der Destillationsrückstand (100) und ein Trägergas (110) in einen Reaktor (1) eingesprüht werden, wobei der Destillationsrückstand (100) und das Trägergas (110) vertikal nach unten strömen, sodass das monomere Isocyanat teilweise bis vollständig verdampft und so ein getrockneter, von monomerem Isocyanat weitgehend bis vollständig befreiter Rückstand (120) und ein monomeres Isocyanat umfassender Strom (130) erhalten werden.

2. Verfahren nach Anspruch 1, bei dem der das monomere Isocyanat umfassende Strom 130 in einem Seitenabzug des Reaktors 1 und der Rückstand 120 dem Reaktor 1 unterhalb dieses Seitenabzugs entnommen werden.

3. Verfahren nach Anspruch 1 oder 2, bei dem die Temperatur des Destillationsrückstands (100) beim Einsprühen in den Reaktor (1) von 20 °C bis 300 °C und die Temperatur des Trägergases (110) beim Einsprühen in den Reaktor (1) von 150 °C bis 500 °C beträgt.

4. Verfahren nach einem der Ansprüche 1 bis 3, bei dem der Destillationsrückstand (100) unter einem absoluten Druck von 1,0 bar bis 300 bar mittels einer Druckdüse in den Reaktor 1 eingesprüht wird.

5. Verfahren nach einem der Ansprüche 1 bis 4, bei dem in den Reaktor (1) zusätzlich ein Trennmittel ausgewählt aus der Gruppe bestehend aus Talkum, Kreide, anorganischen Pigmenten und vollständig durchpolymerisiertem Rückstand eingesprüht wird.

6. Verfahren nach einem der Ansprüche 2 bis 5, bei dem im Reaktor (1) unterhalb des Seitenabzugs für den das monomere Isocyanat umfassenden Strom (130) eine Wirbelschicht aus einem von unten zugeführten Fluidisiergas und Feststoffpartikeln eingestellt wird.

7. Verfahren nach einem der Ansprüche 1 bis 6, bei dem der Destillationsrückstand (100) in Schritt b) erhalten wird durch Aufkonzentrierung des Sumpfproduktes eines Destillationsschritts in einem Dünnschichtverdampfer, Kletterverdampfer, Fallfilmverdampfer, Langrohrverdampfer, Wendelrohrverdampfer, Zwangsumlaufentspannungsverdampfer oder Schaufeltrockner oder in einer Kombination dieser Apparate, wobei eine Gasphase umfassend monomeres Isocyanat und der Destillationsrückstand (100) als flüssige Phase erhalten werden.

8. Verfahren nach einem der Ansprüche 1 bis 7, bei dem das Isocyanat ausgewählt ist aus der Gruppe bestehend aus Toluylendiisocyanat, Diphenylmethandiisocyanat, 1,6-Hexamethylendiisocyanat, Isophorondiisocyanat und Diisocyanatodicyclohexylmethan.

9. Verfahren nach Anspruch 8, bei dem das Isocyanat Toluylendiisocyanat ist.

## Claims

1. Process for the preparation of an isocyanate, comprising the following steps.
a) phosgenation of a primary amine to obtain a crude process product comprising the corresponding isocyanate,
b) working up of the crude process product obtained under a), wherein the working up comprises at least one distillation step in which monomeric isocyanate, as the distillate, and a distillation residue (100) comprising monomeric isocyanate are obtained,
c) working up of the distillation residue (100) obtained under b), wherein the distillation residue (100) and a carrier gas (110) are sprayed into a reactor (1), the distillation residue (100) and the carrier gas (110) flowing vertically downwards, so that the monomeric isocyanate vaporizes partly to completely and a dried residue (120) which has been largely to completely freed from monomeric isocyanate and a stream (130) comprising monomeric isocyanate are obtained in this way.

2. Process according to Claim 1, in which the stream 130 comprising the monomeric isocyanate is removed in a lateral take-off of the reactor 1 and the residue 120 is removed from the reactor 1 below this lateral take-off.

3. Process according to Claim 1 or 2, in which the temperature of the distillation residue (100) when sprayed into the reactor (1) is from 20 °C to 300 °C and the temperature of the carrier gas (110) when sprayed into the reactor (1) is from 150 °C to 500 °C.

4. Process according to one of Claims 1 to 3, in which the distillation residue (100) is sprayed into the reactor 1 under an absolute pressure of from 1.0 bar to 300 bar by means of a pressure nozzle

5. Process according to one of Claims 1 to 4, in which a release agent chosen from the group consisting of talc, chalk, inorganic pigments and completely polymerized residue is additionally sprayed into the reactor (1)

6. Process according to one of Claims 2 to 5, in which a fluidized bed of a fluidizing gas fed from the bottom and solid particles is established in the reactor (1) below the lateral take-off for the stream (130) comprising the monomeric isocyanate.

7. Process according to one of Claims 1 to 6, in which the distillation residue (100) in step b) is obtained by concentration of the bottom product of a distillation step in a thin film evaporator, climbing film evaporator, falling film evaporator, long tube evaporator, helical tube evaporator, forced circulation flash evaporator or paddle drier or in a combination of these apparatuses, a gas phase comprising monomeric isocyanate and the distillation residue (100), as a liquid phase, being obtained

8. Process according to one of Claims 1 to 7, in which the isocyanate is chosen from the group consisting of toluylene-diisocyanate, diphenylmethane-diisocyanate, 1,6-hexamethylene-diisocyanate, isophorone-diisocyanate and diisocyanatodicyclohexylmethane.

9. Process according to Claim 8, in which the isocyanate is toluylene-diisocyanate

## Revendications

1. Procédé de fabrication d'un isocyanate, comprenant les étapes suivantes.
a) la phosgénation d'une amine primaire pour obtenir un produit de procédé brut comprenant l'isocyanate correspondant,
b) le traitement du produit de procédé brut obtenu en a), le traitement comprenant au moins une étape de distillation lors de laquelle de l'isocyanate monomère se forme en tant que distillat et un résidu de distillation (100) comprenant de l'isocyanate monomère,
c) le traitement du résidu de distillation (100) obtenu en b), le résidu de distillation (100) et un gaz vecteur (110) étant injectés dans un réacteur (1), le résidu de distillation (100) et le gaz vecteur (110) s'écoulant verticalement vers le bas de sorte que l'isocyanate monomère s'évapore partiellement à complètement et qu'un résidu sec (120) essentiellement à complètement débarrassé d'isocyanate monomère et un courant (130) comprenant de l'isocyanate monomère soient obtenus.

2. Procédé selon la revendication 1, dans lequel le courant 130 comprenant l'isocyanate monomère est soutiré par une sortie latérale du réacteur 1 et le résidu 120 en dessous de cette sortie latérale du réacteur 1

3. Procédé selon la revendication 1 ou 2, dans lequel la température du résidu de distillation (100) lors de l'injection dans le réacteur (1) est de 20 °C à 300 °C, et la température du gaz vecteur (110) lors de l'injection dans le réacteur (1) est de 150 °C à 500 °C.

4. Procédé selon l'une quelconque des revendications 1 à 3, dans lequel le résidu de distillation (100) est injecté dans le réacteur 1 à une pression absolue de 1,0 bar à 300 bar au moyen d'une buse sous pression

5. Procédé selon l'une quelconque des revendications 1 à 4, dans lequel un agent de séparation choisi dans le groupe constitué par le talc, la craie, les pigments organiques et un résidu complètement polymérisé est injecté en outre dans le réacteur (1).

6. Procédé selon l'une quelconque des revendications 2 à 5, dans lequel un lit fluidisé constitué par un gaz de fluidisation introduit par le bas et des particules solides est mis en place dans le réacteur (1) en dessous de la sortie latérale pour le courant (130) comprenant l'isocyanate monomère.

7. Procédé selon l'une quelconque des revendications 1 à 6, dans lequel le résidu de distillation (100) est obtenu à l'étape b) par concentration du produit de fond d'une étape de distillation dans un évaporateur à couche mince, un évaporateur montant, un évaporateur à film tombant, un évaporateur à tube long, un évaporateur à hélice, un évaporateur à détente et à circulation forcée ou un évaporateur à pale, ou dans une combinaison de ces appareils, une phase gazeuse comprenant l'isocyanate monomère et le résidu de distillation (100) étant obtenus sous la forme d'une phase liquide

8. Procédé selon l'une quelconque des revendications 1 à 7, dans lequel l'isocyanate est choisi dans le groupe constitué par le diisocyanate de toluylène, le diisocyanate de diphénylméthane, le diisocyanate de 1,6-hexaméthylène, le diisocyanate d'isophorone et le dnsocyanatodicyclohexylméthane

9. Procédé selon la revendication 8, dans lequel l'isocyanate est le diisocyanate de toluylène.
